# EUROPEAN PATENT APPLICATION

(11) **EP 1 762 229 A2**
(43) Date of publication of application: **14.03.2007**
(21) Application number: 06254729.4
(22) Date of filing: 11.09.2006
(51) Int. Cl.: A61K 9/12

(54) **Sprayable personal lubricant**

(30) Priority: 12.09.2005 US 716230 P; 13.04.2006 US 403523
(71) Applicant: Mcneil-PPC, Inc, Skillman, NJ 08558 (US)
(72) Inventor: Ahmad, Nawaz, Monmouth Junction, NJ 08852 (US); Cui, Cheng-Ji, Pennington, NJ 08534 (US); Eknoian, Michael, Warren, NJ 07059 (US); Walters, Russell, Philadelphia, PA 19106 (US); Librizzi, Joseph, Hillsborough, NJ 08844 (US); Morscherosch, Michael, Doylestown, PA 18901 (US); Ison, Bryant, Lawrenceville, NJ 08648 (US); Mohary, Stephen J., Pennington, NJ 08534 (US)
(74) Representative: Fisher, Adrian John

(57) **Abstract**

This invention relates to personal lubricant compositions that are capable of being sprayed to a targeted area while maintaining lubricity.

## Description

This application is a non-provisional application based upon Provisional Patent Application Serial No. 60/716,230 filed September 12, 2005 and hereby incorporates herein the subject matter of that application.

### Field of the Invention

The present invention relates to a composition useful for personal lubrication during intimate contact that is applied by atomization. In particular, the compositions are sprayed onto a defined body target area.

### Background of the Invention

Personal lubrications for intimate contact are well known. Typically, personal lubricants are marketed as liquids, jellies, gels or suppositories. Examples of such products include K-Y® Jelly, Astroglide®, K-Y® Liquid, K-Y® Ultragel^{™}. More recently K-Y® Warming Liquid was introduced to the marketplace. K-Y ® Warming Liquid is a water soluble, anhydrous composition that warms on contact while providing lubrication.

Most of the commercially available personal lubricant products are used by first applying to the hand or fingers of the user and then to the intimate area. This can be undesirable and messy. Additionally, some individuals are adverse to applying a personal lubricant directly to the genital regions.

One method to deliver a liquid hands-free is through spraying. Spraying a composition can be accomplished in one or more ways. For example, there exist two products from Durex sold in a spray pump dispenser. When dispensed, the products, Play Warmer Lubricant and Play Tingling Lubricant, come out of the nozzle in a viscous stream or single line, the direction of which is controlled by gravitational force. The stream of lubricant does not extend beyond a short distance, making it difficult to dispense to a specific target area. Additionally, the stream that is sprayed out is not atomized and does not result in a relatively uniform layer of lubricant over a target area. Furthermore, the pumping mechanism does not change the properties of the composition when dispensed.

There are also commercially available oils, lubricants and food products that reside in spray containers that utilize atomization. For example, WD-40^{®}, a household lubricant containing petroleum distillates (commercially available from the WD-40 Company of California), is an aerosol containing carbon dioxide propellant that can be sprayed in a wide spray pattern. This product also has a straw-type attachment, which allows it to be delivered to a specific site.

Another spray pump dispensing device that is capable of atomizing a composition contains a swirl chamber that breaks up the composition into minute particles or into a fine spray, which is then expelled. Examples of products that may be atomized are olive oil sprays, artificial oleo spreads, hair care products, mechanical lock lubricants, sun care skin products and massage oils.

However, not all liquids are capable of being sprayed. This has been a particular challenge in the area of personal lubricants. Typically, personal lubricants have a high viscosity, resulting in a much thicker solution than is generally thought of as being capable of being sprayed. Additionally, some personal lubricants are not liquids but are gels and jellies. Other products which require both sprayability and lubricity may be conveniently made in accordance with the compositions and methods of this invention, for example, hair sprays or skin moisturizers.

Lowering the viscosity of such personal lubricant compositions by diluting the composition with liquid, however, generally causes the composition to lose lubricity.

Therefore, there exists a need for a sprayable composition for personal lubrication use that results in a controlled delivery to a defined target area.

### BRIEF DESCRIPTION OF THE DRAWINGS

FIG. 1 illustrates the conical spray pattern of compositions of this invention.

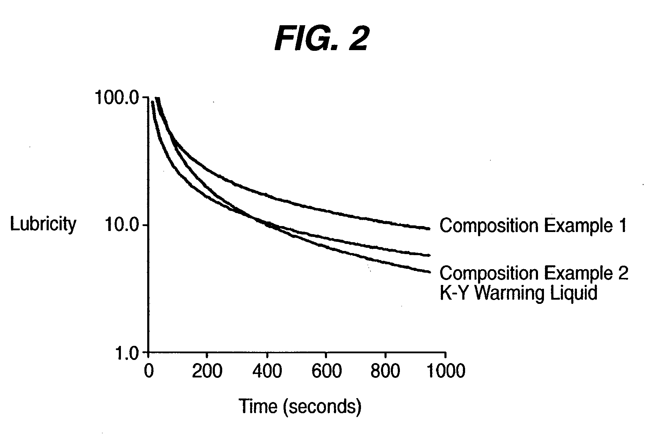

FIG. 2 is a graph illustrating a comparison of lubricity of Composition Example 1 and Example 2 of the invention with K-Y® Warming Liquid.

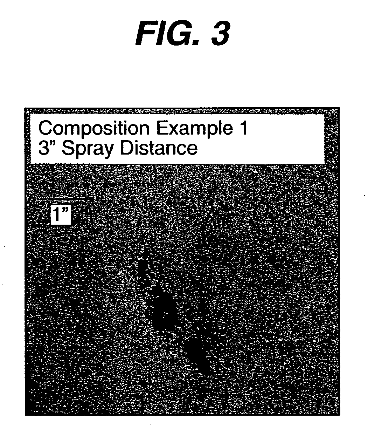

FIG. 3 is a photograph of a preferred spray pattern and spray area covered by the composition 1 of the invention when it is applied from a distance of 3 inches.

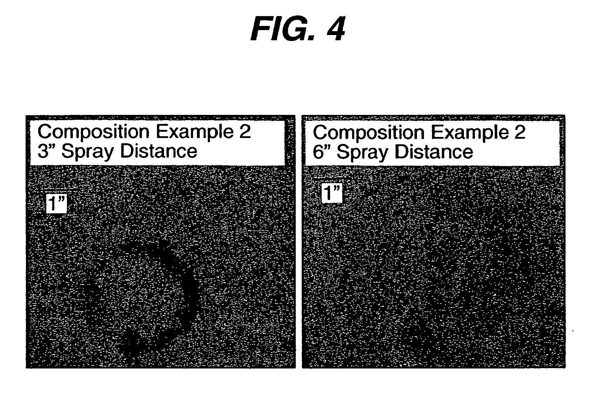

FIG. 4 is a photograph of a preferred spray pattern and spray area covered by Example 2 of the invention from a distance of 6 inches and 3 inches.

### SUMMARY OF THE INVENTION

We have discovered that, unexpectedly, imparting certain physical characteristics to personal lubricant compositions endows them with the capability of being sprayable while retaining sufficient lubricity to be used as a personal lubricant.

Preferably, the personal lubricant compositions of this invention have a lubricity of at least about 8 and exhibit lubricious behavior after spraying and when applied to a surface.

### DETAILED DESCRIPTION OF THE INVENTION

As used herein, the term "spray stream" refers to a spray pattern wherein the composition is disposed within approximately a single line. The line may initially be straight, but may be caused to curve in accordance with gravitational force exerted upon the stream. The composition does not radiate outward from the line.

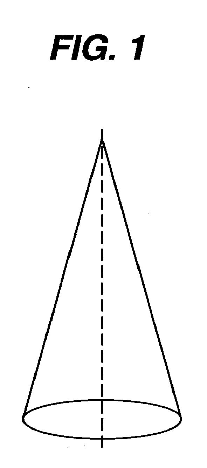

As used herein, the terms "target spray", "target sprayable" are defined to mean that the composition may be dispensed from a nozzle in a conical-shaped pattern when viewed from the side. This excludes all types of sprays that deliver in a stream. In one embodiment, the target spray dispenses from the nozzle orifice such that the droplets radiate outward. If a central axis were to be drawn perpendicular from the nozzle orifice, the droplets would radiate outward about such axis at an angle greater than zero degrees but less than 180 degrees. One example of this pattern is set forth in **FIGURE 1.**

The target spray preferably results in a "filled pattern" upon the target area. As used herein, the term "filled pattern" means that the composition of the target spray is distributed and coats the at least a portion of the defined target area and not just the outer edges, which results in a "halo" appearance. While the defined target area need not have a defined shape, it is possible by optimizing the sprayer and spray nozzle to have a defined target area of, for example, a diamond. Other embodiments such as circles, ovals and the like are possible.

The intent of coating the defined target area with a filled spray pattern is to ensure that the application of the composition may be controlled and applied to a desired target area. This would prevent the composition from being applied to an area that is not desired by the individual using the product, i.e., bedding, clothes, flooring, furniture or other portions of anatomy.

The term "mist" is used herein to refer to a spray pattern that does not have clearly defined outer boundaries.

Unexpectedly, the compositions of this invention have relatively high lubricity even after having been sprayed. In one embodiment, the defined target area has an area of about 0.75 to about 3 square inches. In another embodiment, the defined circular target area is 0.5 to about 2.5 square inches.

The compositions of this invention may be gels or viscous liquids that exhibit what is known as "Non-Newtonian" behavior. For Non-Newtonian fluids, the viscosity parameter is a function of shear rate. Many Non-Newtonian liquids exhibit the behavior of "shear thinning". Some liquids at rest appear to behave like solids until shear stress exceeds a certain value, called the yield stress, after which they flow readily.

Under the stress of being drawn up a dip tube or being expelled under pressure, the compositions of this invention should preferably exhibit shear thinning behavior and will then be capable of being sprayed as a liquid. Unexpectedly, the compositions of this invention have relatively high lubricity even after having been sprayed.

In order to obtain the filled pattern, it may be necessary to spray the composition from a predetermined distance from the target area. For example, in one embodiment, a user may position the spray nozzle approximately 1.5 to 4 inches from the body. In another embodiment, the user may hold the spray nozzle approximately 2 to 3 inches from the body. Additionally, the spray container may be configured to be sprayed in an upright manner or upside down. For example, a female user may prefer to invert the container while dispensing the contents to her perineal area more conveniently.

As used herein, the phrase "personal lubrication" means those types of compositions that supply lubrication during personal or sexual relations. The personal lubrication of this invention may be applied to the vagina, vaginal area, perineum, anal area, penis, or oral cavity. In the event that manual stimulation or penetration is desired, the composition may be applied to the hands or fingers. The composition may also be applied to devices such as medical devices, gloves, or sexually-related devices such as vibrators, sexual aids, and the like. The personal lubricant may also contain flavors or fragrances to impart sensory variety to the composition.

The composition utilized in this invention may be any composition that is sprayable and lubricating in accordance with this invention. In one embodiment, the sprayable composition may be an aqueous composition.

If the compositions of this invention are to be utilized in conjunction with a latex condom, however, they should not contain oil or other petroleum products or other components that would tend to degrade such condoms.

In one embodiment, the compositions of this invention contain at least one polyhydric alcohol, which is water-soluble, and a water-soluble polymer.

In one embodiment, the composition of the present invention contains at least one polyhydric alcohol. The at least one polyhydric alcohol portion may contain propylene glycol, polyethylene glycol, sorbitol or a combination thereof. Polyethylene glycol may be selected ranging in molecular weight of from 300 to about 1450. The polyhydric alcohol portion of the compositions of this invention should make up from about 5 to about 90% by weight of the composition. More preferably the compositions of this invention should contain a combination of two or more polyhydric alcohols. Most preferably, the polyhydric portion of the composition should contain glycerin and propylene glycol. Preferably there should be from about 5 to 50% by weight of propylene glycol and 2 to about 40% by weight of glycerin.

Preferably, the water-soluble polymer is a water-soluble polyacrylic acid polymer may be a Carbopol polymer, such as Carbopol 940, Carbopol 934P, Carbopol 974P, Carbopol 980 or Carbopol 981. In one embodiment, the polyacrylic acid polymer is a pharmaceutical grade Carbopol such as Carbopol 974P. The water-soluble polymer ingredient may also preferably be a polyvinylpryolidone or vinyl acetate/crotonic acid polymer such as Aristoflex. The water-soluble polymer portion of the compositions of this invention should make up from about 0.2% to about 2% by weight of the total composition.

In one embodiment, the acrylic acid polymer is neutralized by a base. In one embodiment, the base is an inorganic base such as sodium hydroxide, potassium hydroxide or ammonium hydroxide. In one embodiment, the inorganic base is potassium hydroxide. The inorganic base should make up from about 0.1 to about 1.5% by weight of the total composition. Additionally, an inorganic base may be used to adjust the pH of the composition to be compatible with the vaginal, rectal or oral membranes. Of course, any other physiologically acceptable base may also be utilized in this manner.

A preservative may be important for use in the products of this invention, in order to preserve the stability of the compositions of this invention and to prevent the growth of microorganisms therein. The preservative portion of the compositions of this invention may be one or more known preservatives, such as methylparaben, benzoic acid, sorbic acid, gallic acid or propylparaben. From about 0.05% to about 0.75% by weight preservative should be used.

Other ingredients which may be included in the compositions of this invention preferably include those set forth in Such warming personal lubricants are described in, for example, U.S. Patent No. 7,005,408 as well as in U.S. Patent Applications Serial Nos. 10/390,511 filed March 17, 2003, 10/389,871, filed March 17, 2003, 10/696,939, filed October 30, 2003, 10/697,353, filed October 30, 2003, 10/697,838, October 30, 2003, 10/847,082, May 17, 2004 and 10/847,083, filed May 17, 2004, which are hereby incorporated herein by reference and in copending patent applications Serial No. (Attorney Docket No. PPC-5232NP) and (Attorney Docket No. PPC-5210NP) the subject matter of which is incorporated herein by reference, including antioxidants such as ascorbic acid, butylated hydroxytoluene and tocopherol.

Other ingredients may be used to make the composition benzoic acid, potassium sorbate, sodium benzoate, propylparaben, sodium, bisulfite, sassafras oil, sodium metabisulfite, sorbic acid, thimersal, maleic acid and propyl gallate.

The compositions may also include organic acids such as benzoic acid, citric acid, linolic acid, oxalic acid, ketoglutaric acid, tannic acid, humic acid, glycolic acid, gallic acid and malic acid.

Water, which makes up the remaining portion of the compositions of this invention functions to provide appropriate consistency, viscosity, sprayability and lubricity of the compositions.

A preservative may be added to the compositions of this invention in order to curb microbial growth. A preservative may be selected from preservatives known to those of skill in the art, including, but not limited to, one or more of the following: methylparaben, benzoic acid, sorbic acid, gallic acid, propylparaben or the like. The preservative may be present in the compositions of this invention in an amount from about 0.01% to about 0.75% by weight of the composition.

As defined earlier, the sprayable composition of this invention may be used as personal lubricants and/or moisturizers. The compositions of this invention may also possess a sweet and pleasant taste, which is of particular benefit when these compositions are used orally. Such personal lubricants are useful in facilitating sexual intercourse and serve to enhance intimacy in sexual activities.

Yet other embodiments of the compositions of this invention are compositions that may include local anesthetics. The local anesthetics may preferably include, but are not limited to, benzocaine, lidocaine, dibucaine, benzyl alcohol, camphor, resorcinol, menthol and diphenylhydramine hydrochloride and the like.

Compositions of the invention may also include plant extracts such as aloe, witch hazel, chamomile, hydrogenated soy oil and colloidal oatmeal, vitamins such as vitamin A, D or E and corticosteroids such as hydrocortisone acetate.

### Lubricity

The compositions of the present invention provide personal lubrication. Personal lubricants substantially prevent irritation, which may result due to friction during sexual activity. As an example, some post-menopausal women find sexual intercourse painful due to dryness of the vagina. Such a condition may also be the result of other origins including female sexual dysfunction. The use of a personal lubricant helps to overcome this condition.

Lubricity may be measured using the following test method known herein as the ***"Ahmad Procedure"*** which was described in U.S Patent No. 6,139,848. Briefly, the test method measures the amount of force required to move one surface relative to another while under weight pressure (the two surfaces being horizontal to each other). A weight or pressure can be applied on the upper moving surface. The test composition, in this case, the test lubricants, work by reducing the friction between the two surfaces. From this force and weight, a coefficient of friction value for a lubricant can be calculated. The coefficient of friction is inversely proportional to the lubricity of a product and is known as "relative lubricity". Relative lubricity can be calculated from the coefficient of friction data by dividing the numeral one by the corresponding coefficient of friction value.

An instrument, namely Coefficient of Sliding Friction Rig adapted to a Texture Analyzer, marketed by Texture Technologies Corp., 18 Fairview Road, Scarsdale, N.Y. was used for determining relative lubricity of several lubricant products in comparison with that of the compositions of this invention. The equipment contains a platform having a friction sledge attached to a load cell which is constrained to slide across the platform over which a test sample is applied. Load is provided by a 430 g precision weight positioned centrally over the sledge. This arrangement offers the advantage of measuring coefficient of sliding friction in both directions such that data for the "push" and the "pull" phases of the test can be generated over a fixed period of time. For the examples set forth below, it was possible to generate coefficient of friction data for an extended period of five (5) minutes. In making the measurements for the examples, a non-lubricated condom was mounted over the sledge, a thin film of the lubricant sample was applied over the fixed platform and coefficient of sliding friction readings were recorded over a five-minute period while the friction sledge went back and forth from the starting point. The coefficient of friction data, therefore, has negative (-) sign during the "push" phase and positive (+) sign during the "pull" phase of the experiment. The coefficient of friction data for the baseline, with no product applied to the condom was also generated for comparison. Texture Analyzer TA-XT2I (SID 41) was utilized for the test, having a Plexiglas^{™} plate 3" x 4" x 3/8" in size, a 430 g weight and a 6.0 mil Bird Applicator. The substrate used was a polyethylene/foil liner and a Trojans^{®} non-lubricated condom. The texture analyzer settings were as follows: Test Mode and Option, Measure Force In tension, Cycle Until Count, Trigger, Type-Button, Stop Plot at--Trigger Return, Brea--Detect off, level. The pre-test speed was 0 mm/s, the test speed was 2.0 mm/s, the post test speed was 0.0 mm/s and the distance traveled was 40.0 mm. The test was run for 300 seconds. The PE/foil liner was glued to the aluminum base or platform of the Texture Analyzer. The Plexi-glas^{™} sled was covered with the condom, a 6.0 mil film of test product was cast onto the liner and the 430 g weight placed on the center of the sled.

Preferably, the lubricity range for the compositions of this invention as determined using the Ahamd procedure should be at least about 8 for the duration of between about 100 and about 900 seconds, more preferably, more than about 30 for the duration of between about 100 and about 900 seconds.

### Viscosity

In order for the composition to be sprayable, the composition must have a certain viscosity, which allows the composition to be sprayed. It has been found that while achieving the appropriate viscosity, maintaining lubricity is equally important.

The composition may be sprayed through a pump spray or a spray valve or an actuator adapted to a container. The compositions of this invention may also be dispensed using an aerosol spray device, preferably one that does not employ propellant in the spray composition.

The viscosity must be such that the composition can be drawn into and up through the delivery mechanism of the sprayer. This may include a dip tube, into an actuator and a spray orifice. In one embodiment, the viscosity of the composition is in the range of 250 to about 2000 cps as measured by a Brookfield Instrument with an RVT spindle at a speed of 20 rpm at 25° C. In a more preferred range, the viscosity is in the range of from about 500 to about 1000 cps.

### Spray Mechanisms

In one embodiment, the spray mechanism useful with this invention is a spray pump-actuator combination.

In spray pumps having a swirl chamber, the composition is typically drawn up into the chamber by a dip tube. Once inside the swirl chamber, the composition breaks up into minute particles or to a fine spray, which is then expelled.

Once the spray pump has been primed, the composition is delivered to the defined target area in predetermined amounts. In one embodiment, the pump required at least two strokes to prime. In another embodiment, the pump required at least three strokes to prime. During priming, the composition is drawn up the dip tube, into the swirl chamber, through the actuator and eventually expelled. In one embodiment, the spray pump delivers 120 to 160 µL of composition per a single stroke into the defined target area.

In order to be sprayable, the compositions of this invention should be capable of being atomized into particles having a size distribution as follows, given for the 10^{th}, 50^{th}, and 90^{th} percentiles:

| Distance | | | Particle Size (µm) |
|---|---|---|---|
| | D[v,0.1] | D[v,0.5] | D[v,0.9] |
| 3" | 417 | 40 | 119 |
| 6" | 235 | 40 | 81 |

Preferably, particle size at 3" should be at least about 400 µm in the 10^{th} percentile, at least about 40 µm in the 50^{th} percentile and at least about 100 µm in the 90^{th} percentile and at 6", at least about 200 µm in the 10^{th} percentile, at least about 40 µm in the 50^{th} percentile and at least about 75 µm in the 90^{th} percentile.

In one embodiment, the spray pump may be a two-way pump, that is, the composition contained within the pump can be dispensed from an upright or inverted configuration such that the user can spray "upside down".

Preferably, the compositions and methods of this invention enable the compositions to be dispensed from a location remote from the target area with minimal dispersion to undesired areas. For example, the compositions may be sprayed at a target area with reasonable accuracy from a distance of from at least about three to at least about twelve inches or further.

The compositions of this invention are preferably propelled out of the dispenser at a pressure which is high enough to cause shear thinning and that will counteract gravitational force. This preferably occurs whether or not the compositions of this invention are aerosolized by the pumping device utilized to dispense them. The compositions of this invention may be propelled from a pump spray or an aerosol spray, which dispenses a continuous spray rather than a "batch" spray, depending upon whether the pump must be primed. The methods of this invention also provide the user with a controlled dose of composition delivered to a target area if the compositions are dispensed using a primed pump.

The methods of this invention include methods of applying personal lubricants by placing a composition of this invention in a pump spray mechanism and dispensing the composition to a target area from a distance of at least three inches. Such compositions may be applied to mucous membranes, including oral, nasal, or vaginal membranes. The method of this invention may also include applying the compositions of this invention to external genitalia prior to or during intercourse in order to provide personal lubrication during intercourse.

The following examples serve to illustrate the compositions and methods of this invention. However, they are not presented in order to limit the scope of the invention in any way.

### Inventive Examples 1-5 were prepared as follows:

The compositions were or may be prepared by adding Carbopol 974P to water and dispersing it using a high-speed mixer. The dispersion was then heated to a temperature of between 50 to 60°C and benzoic acid, propylene glycol, and glycerin were added. The contents were mixed until a clear solution is obtained. In a separate container, sodium hydroxide solution is prepared by adding sodium hydroxide pellets to water, previously weighed out. The sodium hydroxide solution was added to the main batch while mixing until a clear smooth liquid or gel was obtained. The contents were cooled to room temperature.

### Inventive Example 1

| Ingredient | % w/w |
|---|---|
| Propylene Glycol | 35.00 |
| Glycerin | 15.00 |
| Benzoic Acid | 0.20 |
| Carbopol 974P | 0.60 |
| Potassium Hydroxide | 0.12 |
| Purified Water | 49.08 |
| Total | 100.00 |

### Inventive Example 2

| Ingredient | % w/w |
|---|---|
| Propylene Glycol | 20.00 |
| Glycerin | 10.00 |
| Benzoic Acid | 0.20 |
| Carbopol 974P | 0.40 |
| Potassium Hydroxide | 0.12 |
| Purified Water | 69.28 |
| Total | 100.00 |

### Inventive Example 3

| Ingredient | % w/w |
|---|---|
| Propylene Glycol | 35.00 |
| Polyethylene Glycol 400 | 15.00 |
| Benzoic Acid | 0.20 |
| Carbopol 974P | 0.60 |
| Potassium Hydroxide | 0.12 |
| Purified Water | 49.08 |
| Total | 100.00 |

### Inventive Example 4

| Ingredient | % w/w |
|---|---|
| Propylene Glycol | 50.00 |
| Benzoic Acid | 0.20 |
| Carbopol 974P | 0.60 |
| Potassium Hydroxide | 0.12 |
| Purified Water | 49.08 |
| Total | 100.00 |

### Inventive Example 5

| Ingredient | % w/w |
|---|---|
| Glycerin | 30.00 |
| Benzoic Acid | 0.20 |
| Carbopol 974P | 0.60 |
| Potassium Hydroxide | 0.12 |
| Purified Water | 69.08 |
| Total | 100.00 |

### Comparative Examples 1-3

Relative lubricity data presented in FIG. 1 was calculated from the coefficient of friction data generated using the Ahmad Method as described above. The lubricants tested were the compositions as detailed in Inventive Examples 1 and 2 and K-Y® Warming Liquid, a commercially available product. As shown, Example 1 overall showed higher lubricity that continued for a longer period of time than K-Y® Warming Liquid. Additionally, Example 2 exhibited similar lubricity to K-Y® Warming Liquid from 0 to 400 seconds at which time, Example 2 maintained lubricity while K-Y® Warming Liquid decreased.

Table 1 shows the sprayablity of commercial products and Examples 1 and 2 as described above.

| Sample | Form | Sprayable Composition* (yes or no) |
|---|---|---|
| Commercial Product 1 | Liquid | No |
| Commercial Product 2 | Gel | No |
| Commercial Product 3 | Liquid | No |
| Commercial Product 4 | Liquid | No |
| Commercial Product 5 | Liquid | No |
| Commercial Product 6 | Liquid | No |
| Commercial Product 7 | Gel | No |
| Commercial Product 8 | Liquid | No |
| Commercial Product 9 | Liquid | No |
| Commercial Product 10 | Liquid | No |
| Commercial Product 11 | Liquid | No |
| Commercial Product 12 | Liquid | No |
| Commercial Product 13 | Liquid | No |
| Commercial Product 14 | Liquid | No |
| Example 1 | Liquid | Yes |
| Example 2 | Liquid | Yes |

| | | |
|---|---|---|
| *Sprayable composition denotes a filled pattern upon the target area. Commercial Product 1: K-Y® Liquid^{™} (Personal Products, Skillman, N.J.) Commercial Product 2: K-Y® Ultragel^{™} (Personal Products, Skillman, N.J.) Commercial Product 3: Astroglide (BioFilm, Inc., Vista, CA.) Commercial Product 4: WET Original (WET Formula International, North Hollywood, CA.) Commercial Product 5: WET Light (WET Formula International, North Hollywood, CA.) Commercial Product 6: ForPlay (Trimensa Pharmaceuticals, Newbury Park, CA.) Commercial Product 7: Vagisil Intimate Moisturizer (COMBE Incorporated, White Plains, N.Y.) Commercial Product 8: Aqua Lube (Mayer Laboratories, Inc., Oakland, CA.) Commercial Product 9: PROBE (Davryan Laboratories, Inc., Portland, OR.) Commercial Product 10: EROS (CDC Distribtion Center, Fleishstraube, Germany) Commercial Product 11: play (Durex Consumer Products, Inc., Norcross, GA.) Commercial Product 12: SYLK (Geneva Marketing, Auckland, New Zealand) Commercial Product 13: TROJAN Crystal Clear Liquid (Church & Dwight Co., Inc., Princeton, N.J.) Commercial Product 14: I-D (Westridge Laboratories, Inc., Newport Beach, CA.) | | |

As shown by the results set forth in Table 1, there are no commercially available lubricant products that deliver the lubricant in a filled pattern upon the target area.

## Claims

1. A composition for application to a mammal, wherein the composition is sprayable and lubricious.

2. A sprayable composition according to claim 1, wherein the composition has a lubricity of at least 8 as determined by the Ahmad procedure.

3. An aqueous sprayable composition according to claim 2, wherein the viscosity of the composition is from about 250 to about 2000 cps.

4. A composition according to claim 1 wherein said composition comprises at least one polyhydric alcohol.

5. A composition according to claim 1 wherein said composition comprises a water soluble acrylic acid polymer.

6. A composition according to claim 1 which sprays a targeted area in a conical pattern.

7. A composition according to claim 1 having a lubricity of at least 8 and a viscosity of from about 250 to about 2000 cps.

8. A method of providing personal lubrication comprising placing a composition according to claim 1 in a pump spray mechanism and dispensing said composition to a target area from a distance of at least three inches.

9. A method of providing personal lubrication comprising dispensing a composition according to claim 1 as a target spray.

10. A method of providing personal lubrication in accordance with claim 9 from a distance of at least three inches.
